# EUROPÄISCHE PATENTANMELDUNG

(11) **EP 0 744 177 A2**
(43) Veröffentlichungstag der Anmeldung: **27.11.1996**
(21) Anmeldenummer: 96107867.2
(22) Anmeldetag: 17.05.1996
(51) Int. Cl.: A61K 35/78

(54) **Substanz von mindenstens einem ätherischen Öl mit einem Provitamin, insbesondere zur Verhütung und Behandlung von Dekubitus**

(30) Priorität: 23.05.1995 DE 19518836
(71) Anmelder: Hartwig, Gisela, 36251 Ludwigsau (DE)
(72) Erfinder: Hartwig, Gisela, 36251 Ludwigsau (DE)
(74) Vertreter: WALTHER, WALTHER & HINZ Patentanwälte

(57) **Zusammenfassung**

Substanz, insbesondere zur Verhütung und Behandlung von Dekubitus, beinhaltend einen Trägerstoff, ein das Zellwachstum anregendes ätherisches Öl, ein die Durchblutung förderndes ätherisches Öl, einen Emulgator zum Bilden einer Emulsion der ätherischen Öle mit dem Trägerstoff und ein Provitamin.

## Beschreibung

Die Erfindung betrifft eine Substanz, insbesondere zur Verhütung und Behandlung von Dekubitus, mit einem Trägerstoff, mindestens einem ätherischen Öl und einem Emulgator zum Bilden einer Emulsion des ätherischen Öles mit dem Trägerstoff und mit einem Pro-Vitamin.

Aus der "Roten Liste 1995", Arzneimittel-Verzeichnis des BPI und VFA, ECV-Editio Cantor AULENDORFF/WÜRD; Nummer 84067, ist unter dem Handelsnamen "Heloekzem noro" eine Heilsalbe bekannt, die neben Pantenol und einem Emulgator auch Ol. Chamomillae enthält.

Aus B. Schurch, "DEUTSCHE MEDIZINISCHE WOCHENSCHAU"119/13 (1994), Seite 485 ist es zur Behandlung von Dekubitus, insbesondere zur Epithelisierung bekannt, neben einer Salbe mit dem Handelsnamen "Solkoseryl" auch ein Hautverstärkungsmittel mit Panthenolum, einem Emulgator und Chamomillae zu verwenden. Aus der AU 630,561 ist eine Creme zur Behandlung von Verbrennungen bekannt, welche neben Chamomillae Öl auch Myrrhe-Öl aufweist.

Alle diese Substanzen setzen als ätherisches Öl Kamillenöl ein, welches in der Hauptsache ein antiseptische Wirkung entfaltet und das Zellwachstum bzw. die Durchblutung der Haut nur geringfügig fördert.

In der Alten- und Krankenpflege kommt es häufig vor, daß bettlägerige Patienten an Dekubitus leiden. Bei dieser Krankheit sind große Teile des Gewebes wundgelegen und können nur schwer abheilen, da die Patienten aufgrund ihrer Krankheit oder allgemeinen Schwäche weiterhin auf den wunden Stellen liegen müssen.

Davon ausgehend liegt der Erfindung die Aufgabe zugrunde, eine Substanz zu schaffen, die dem Dekubitus vorbeugt bzw. seine Heilung beschleunigt.

Als technische Lösung dieser Aufgabe wird eine Substanz vorgeschlagen, die ein erstes, das Zellwachstum anregendes ätherisches Öl, ein zweites, die Durchbltung förderndes ätherisches Öl, ein drittes, antivirales, antibakterielles oder antimykotisches, ätherisches Öl und ein viertes, schmerzstillendes, ätherisches Öl enthält.

Es hat sich gezeigt, daß eine Substanz, bei der ein ätherisches Öl auf Niaouli-Basis, ein ätherisches auf Grapefruit-Basis, eine Bohnenkraut-Öl, ein Lemonengras-Öl, ein Cajeput-Öl oder ein Lawendel-Öl unter Zuhilfenahme eines Emulgators in destilliertem Wasser gelöst ist und die darüber hinaus ein Provitamin aufweist, den Dekubitus von bettlägerigen Patienten erfolgreich heilt. Bei dieser Substanz liegt der Anteil der ätherischen Öle im Gesamt-Volumen zwischen 1 und 10 Volumen-%, vorzugsweise 5 %, während der Anteil des Provitamins am Gesamt-Volumen der Substanz zwischen 3 und 5 Volumen-% liegt.

Durch den Einsatz mehrerer, verschiedener ätherischer Öle ist es möglich, mit ein und derselben Substanz in gleichzeitig unterschiedliche Wirkungen zu realisieren, so daß der Heilungsprozeß beschleunigt wird.

Diese Substanz wird vorzugsweise als Lotion verabreicht und beschleunigt das Zellwachstum und fördert die Durchblutung. Die hierdurch beschleunigte Heilung der Patienten wird allgemein begrüßt.

Durch die Hinzunahme eines Dickungsmittels, vorteilhafterweise Xanthan, wird die Substanz dickflüssig gemacht und kann als Lotion verabreicht werden. Dies hat den Vorteil, daß die die Wunde verklebende Wirkung einer Salbe vermieden wird und die Substanz aber dennoch solange an der entsprechenden wunden Stelle verbleibt, das sie ihre volle Wirkung entfalten kann.

Xanthan hat außerdem den Vorteil, daß es Wasser speichert und so die Wunde lange feucht hält, so daß sich im Bereich der Wunde ein eigenes, feuchtes Mikroklima ausbildet.

Es ist weiterhin heilungsfördernd, der Substanz Provitamine, insbesondere D-Panthenol, Vitamin E Acetat oder Vitamin A Palmitat hinzufügen. Hierbei sollte der Volumenanteil des D-Panthenol 16 Volumen-%, der Volumenanteil Vitamin E Acetat 8 Volumen-% und der Volumenanteil des Vitamin A Palmitat 0,1 Volumen-% nicht überschreiten.

In einer besonders bevorzugten Ausführungsform wird die erfindungsgemäße Substanz dadurch gebildet, daß 1 - 8 Volumen-% LV 41 Emulgator, 0,2 - 1 Volumen-% Niaouli-Öl, 0,2 - 2 Volumen-% Grapefruit-Öl, 0,1 - 1 Volumen-% Bohnenkraut-Öl, 0,2 - 2 Volumen-% Lemonengras-Öl, 0,2 - 2 Volumen-% Cajeput-Öl, 0,2 - 2 Volumen-% Lawendel-Öl, 0,05 - 0,4 Volumen-% Xanthan, 2 - 16 Volumen-% D-Panthenol, 1 - 8 Volumen-% Vitamin E Acetat und 0,1 - 1 Volumen-% Vitamin A Palmitat hinzugemischt werden, wobei zum Schluß destilliertes Wasser hinzugegeben wird.

Mit dieser Substanz wurden bisher die besten Heilungserfolge erzielt.

### Erstes Ausführungsbeispiel:

Substanz mit destilliertem Wasser als Trägermittel, dem ätherisches Niaouli-Öl und ätherisches Grapefruit-Öl unter Zuhilfenahme eines Emulgators hinzugefügt wurden, wobei der Volumenanteil der Summe der ätherischen Öle zwischen 1 und 10 %, vorzugsweise 5 % des Gesamtvolumes liegt. Darüber hinaus sind dem destilliertem Wasser 3 - 5 Volumen-% D-Panthenol zugesetzt.

### Zweites Ausführungsbeispiel:

Substanz zusammengesetzt aus
- 0,2 - 1: Vol.% Niaouli-Öl,
- 0,2 - 1: Vol.% Grapefruit-Öl,
- 0,1 - 0,5: Vol.% Bohnenkraut-Öl,
- 0,2 - 1: Vol.% Lemonengras-Öl,
- 0,2 - 1: Vol.% Cajeput-Öl,
- 0,2 - 1: Vol.% Lawendel-Öl,
- 1 - 4: Vol.% LV 41 Emulgator,
- 0,05- 0,2: Vol.% Xanthan,
- 2 - 8: Vol.% D Pathenol,
- 1 - 4: Vol.% Vitamin E Acetat,
- 0,1 - 0,6: Vol.% Vitamin A Palmitat,
und destilliertem Wasser.

## Patentansprüche

1. Substanz, insbesondere zur Verhütung und Behandlung von Dekubitus,
mit einem Trägerstoff, mindestens einem ätherischen Öl und einem Emulgator zum Bilden einer Emulsion des ätherischen Öles mit dem Trägerstoff und mit einem Provitamin,
**dadurch gekennzeichnet**,
daß die Substanz
ein erstes, das Zellwachstum anregendes ätherisches Öl,
ein zweites, die Durchblutung förderndes ätherisches Öl,
ein drittes, antivirales, antibakterielles oder antimykotisches ätherisches Öl, und
ein viertes, schmerzstillendes ätherisches Öl
enthält.

2. Substanz nach Anspruch 1,
**dadurch gekennzeichnet**, daß das Zellwachstum anregende ätherische Öl ein Niaouli-Öl ist.

3. Substanz nach wenigstens einem der vorhergehenden Ansprüche,
**dadurch gekennzeichnet**, daß das die Durchblutung fördernde ätherische Öl ein Grapefruit- und/oder Lemonengras-Öl ist.

4. Substanz nach wenigstens einem der vorhergehenden Ansprüche,
**dadurch gekennzeichnet**, daß das antivirale, antibakterielle oder antimykotische ätherische Öl ein Bohnenkraut-Öl und/oder ein Lemonengras-Öl ist.

5. Substanz nach wenigstens einem der vorhergehenden Ansprüche,
**dadurch gekennzeichnet**, daß das schmerzstillende ätherische Öl ein Cajeput-Öl und/oder ein Lawendel-Öl ist.

6. Substanz nach wenigstens einem der vorhergehenden Ansprüche,
**dadurch gekennzeichnet**, daß das Provitamin ein 1 D Panthenol oder ein Provitamin B ist.

7. Substanz nach Anspruch 6,
**dadurch gekennzeichnet**, daß das Provitamin B ein Vitamin E Acetat oder ein Vitamin A Palmitat ist.

8. Substanz nach wenigstens einem der vorhergehenden Ansprüche,
**dadurch gekennzeichnet**, daß der Emulgator ein LV 41-Emulgator ist.

9. Substanz nach wenigstens einem der vorhergehenden Ansprüche,
**gekennzeichnet durch**
ein Dickungsmittel, insbesondere Xanthan.

10. Substanz nach wenigstens einem der Ansprüche 2 -9, **dadurch gekennzeichnet**, daß das Niaouli-Öl und das Grapefruit-Öl zusammen einen Volumenanteil von 1 - 10 Volumen-% der Substanz ausmachen.

11. Substanz nach wenigstens einem der vorhergehenden Ansprüche,
**dadurch gekennzeichnet**, daß sich die Substanz zusammensetzt aus
0,2 - 2 Vol.% Niaouli-Öl,
0,2 - 2 Vol.% Grapefruit-Öl,
0,1 - 1 Vol.% Bohnenkraut-Öl,
0,2 - 2 Vol.% Lemonengras-Öl,
0,2 - 2 Vol.% Cajeput-Öl,
0,2 - 2 Vol.% Lawendel-Öl,
1 - 8 Vol.% LV 41 Emulgator,
0,05- 0,4 Vol.% Xanthan,
2 -16 Vol.% D-Pathenol,
1 - 8 Vol.% Vitamin E Acetat,
0,1 - 1 Vol.% Vitamin A Palmitat,
und destilliertem Wasser.

12. Substanz nach wenigstens einem der vorhergehenden Ansprüche zur Anwendung in einem Verfahren zur chirurgischen oder therapeutischen Behandlung des menschlichen oder tierischen Körpers, insbesondere zur Verhütung und Behandlung von Dekubitus.

13. Substanz nach wenigstens einem der vorhergehenden Ansprüche,
**dadurch gekennzeichnet**, daß die Substanz als Lotion verabreicht wird.

14. Verwendung einer Substanz, enthaltend einen Trägerstoff, ein Niaouli-Öl, ein Grapefruit und/oder Lemonengras-Öl, einen Emulgator und ein Provitamin, zur Verhütung und Behandlung von Dekubitus.
